(19) **Europäisches Patentamt / European Patent Office / Office européen des brevets**

(11) **EP 1 893 293 B1**

(12) **EUROPEAN PATENT SPECIFICATION**

(45) Date of publication and mention
of the grant of the patent:
**20.08.2008 Bulletin 2008/34**

(21) Application number: **06707404.7**

(22) Date of filing: **28.02.2006**

(51) Int Cl.:
*A61Q 1/14* (2006.01)          *A61Q 19/00* (2006.01)
*A61K 8/02* (2006.01)          *A61K 8/81* (2006.01)
*A61K 8/97* (2006.01)

(86) International application number:
**PCT/EP2006/001973**

(87) International publication number:
**WO 2006/131159 (14.12.2006 Gazette 2006/50)**

(54) **COSMETIC TOWELETTE PRODUCT**

KOSMETIKTUCHPRODUKT

LINGETTE COSMETIQUE

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **06.06.2005 US 145744**

(43) Date of publication of application:
**05.03.2008 Bulletin 2008/10**

(73) Proprietors:
• **Unilever PLC
London
Greater London EC4P 4BQ (GB)**
Designated Contracting States:
**CY GB IE**
• **Unilever N.V.
3013 AL Rotterdam (NL)**
Designated Contracting States:
**AT BE BG CH CZ DE DK EE ES FI FR GR HU IS IT
LI LT LU LV MC NL PL PT RO SE SI SK TR**

(72) Inventors:
• **PADLO, Ewa, Urszula
Norwalk, Connecticut 06851 (US)**
• **MISSO, Luis, R.,
Unilever Home & Personal Care USA
Trumbull,
Connecticut 06611 (US)**

(74) Representative: **Acham, Nicholas Clive
Unilever Patent Group
Colworth House
Sharnbrook
Bedford
MK44 1LQ (GB)**

(56) References cited:
**WO-A-99/06014**          **WO-A-20/04058197**
**US-A1- 2003 206 940**          **US-A1- 2004 096 484**
**US-A1- 2005 031 568**

## Description

BACKGROUND OF THE INVENTION

The invention relates to a cosmetic towelette formed from a water-insoluble substrate such as a non-woven cloth in contact with a cosmetic benefit imparting composition and formulated to maintain consistent towelette color stability.

Cosmetic towelettes have become commercially quite popular. They are handy for cleansing hands and face. Removal of make-up is particularly effective with these products. Wiping cloths offer the advantages of improved impregnated formulation spreadability, abrasive action and a pleasant ergonomic handfeel.

U.S. Patent 6 491 937 B1 (Slavtcheff et al.) is illustrative of the art. Therein is reported a disposable substantially dry foamable water-insoluble flexible web. Consumers at the point of use wet this product to generate a foaming lather.

Another type of wipe product is reported in U.S. Patent 6 294 182 B1 (Znaiden et al.). Therein is disclosed a disposable towelette impregnated with an alpha-hydroxycarboxylic acid. The latter is delivered to the skin for inhibiting formation of facial fine lines and wrinkles.

Common to many of the chemically impregnated towelettes are ingredients which may tend to alter original color of the product. Consequently there is a need for a system which can stabilize or at least minimize color changes.

SUMMARY OF THE INVENTION

A cosmetic towelette product is provided which includes:

(i) a water-insoluble substrate;
(ii) a cosmetic composition in contact with the substrate, the composition including:

(a) from 0.001% to 10% by weight of the composition of a copolymer formed from monomers one of which is a vinyl ester of a $C_{3-20}$ acid;
(b) from 0.000001% to 2% by weight of the composition of a tea extract; and
(c) from 1% to 99.9% by weight of the composition of a cosmetically acceptable carrier.

DETAILED DESCRIPTION OF THE INVENTION

Now it has been found that cosmetic towelettes can be stabilized against changes in color through a combination of a co-polymer formed from monomers one of which is a vinyl ester of a $C_{3-20}$ acid and with a tea extract.

The vinyl ester of a $C_{3-20}$ acid monomer may be, but is not limited to, vinyl propionate, vinyl hexanoate, vinyl octanoate, vinyl decanoate, vinyl isodecanoate, vinyl neodecanoate, vinyl dodecanoate, vinyl hexadecanoate, vinyl octadecanoate, vinyl behenoate and mixtures thereof. Most preferred are vinyl isodecanoate and vinyl neodecanoate. Amounts of this monomer may constitute from 1% to 99%, preferably from 20% to 80%, optimally from 40% to 60% by weight of the copolymer.

One or more other monomers may be present in the copolymer. For purposes of this invention, the term copolymer includes polymers formed from 2, 3, 4, 5 or more different monomers. These other monomers can be selected from acrylic acid, methacrylic acid, ethylacrylate, methyl acrylate, methyl methacrylate, ethyl methacrylate, butyl acrylate, hydroxypropyl methacrylate, hydroxyethyl acrylate, divinyl benzene, vinyl caprolactam, vinyl acetate , maleic acid or anhydride, acrylamide, methacrylamide, $C_{10-30}$ ester of acrylic or methacrylic acids, acryloyldimethyltaurate, styrene, methyl styrene, isoprene, butene, isohexadecane, vinyl chloride, vinyl pyrrolidone, vinyl methyl ether, vinylformamide and combinations thereof, as well as salts of monomers having carboxylate groups. Amounts of the other monomers may range from 0.1% to 99%, preferably from 20% to 80%, optimally from 40% to 60% by weight of the copolymer.

Most preferred is a copolymer with the INCI name of Acrylates/Vinyl Isodecanoate Crosspolymer, commercially available as Stabylen 30® sold by 3V Sigma S.P.A. Stabylen 30® is a copolymer of the ester of vinyl isodecanoate and one or more monomers of acrylic acid, methacrylic acid or one of their simple esters crosslinked with polyalkenyl polyether.

Amounts of the copolymer may range from 0.001% to 10%, preferably from 0.01% to 5%, optimally from 0.1 % to 0.5% by weight of a cosmetic composition.

Tea extract is a term intended to mean extracted components from tea less any solvent. The extracts may either be water soluble or water insoluble and can be carried in a solvent which respectively is hydrophilic or hydrophobic. Water and ethanol are the preferred extract solvents. Amounts of the tea extract may range from 0.000001% to 2%, preferably from 0.0001 % to 1%, optimally from 0.001 % to 0.5% by weight of the cosmetic composition.

References to "tea" herein are to be construed as references to the tea plant, *Camellia Sinensis,* and its products or to equivalent plants, for example *Camellia Assaimica,* or their hybrids, and the products of such equivalent plants, rather than to unrelated herbal tea products such as chamomile and peppermint.

The overwhelming majority of commercial tea products comprise black teas prepared by aeration, and possibly abrasion, of the leaves of the green tea plant conducted for a time sufficient to change the leaf color from green to copper and to intensify their flavor. This is a fermentation process wherein various natural chemical reactions, including enzymatic oxidation, occur. Typical of black tea are fermented leaves such as Darjeeling Assam or Sri Lanka types.

Green tea is another important commercial category. Green teas are produced from the plant without extended air exposure, by merely allowing the plant to wither and dry. This variety includes sencha (middlegrade green tea), *gyokuro* (shaded green tea) or *tencha* (powdered tea) prepared from green tea leaves obtained from the Genus *Camellia,* for example, C. *Sinensis, C. Assamica,* the *Yabukita* variety, or a hybrid thereof.

Another tea is the semi-fermented type which is generally called oolong tea, such as *tekkannon* (Tieguangin), *irotane, ougonkei* (Huangjgui) or *buigancha* (Wuyuyaucha).

Yellow teas are prepared by partial fermentation of the raw green tea product, substantially less than would produce a black tea. White teas are prepared from new buds harvested before they open which are withered and gently dried.

Particularly preferred for the present invention are combinations of *Camellia Sinensis* leaf extract (green tea), white tea leaf extract and Cyclopia Intermedia (Honeybush) Leaf extract. These three tea extracts essentially combine green, white and black tea. The green and white tea extracts may be present in relative weight ratio of 10,000:1 to 1: 10,000, preferably 1,000:1 to 1:1,000, more preferably from 100:1 to 1:100, optimally from 2:1 to 1:2. The ratio of green and white tea to black tea may be from 10,000:1 to 1:10,000, preferably from 1,000:1 to 1:1,000, optimally from 500:1 to 100:1.

Compositions of this invention will also include a cosmetically acceptable carrier. Amounts of the carrier may range from 1 to 99.9%, preferably from 70% to 95%, optimally from 80% to 90%. Among the useful carriers are water, emollients, fatty acids, fatty alcohols, humectants, thickeners and combinations thereof. The carrier may be aqueous, anhydrous or an emulsion. Preferably the compositions are aqueous, especially water and oil emulsions of the W/O or O/W or triplex W/O/W variety. Water when present may be in amounts ranging from 5% to 99%, preferably from 20% to 90%, optimally from 40% to 80% by weight of the composition.

Emollient materials may serve as cosmetically acceptable carriers. These may be in the form of silicone oils, synthetic esters and hydrocarbons. Amounts of the emollients may range anywhere from 0.1% to 30%, preferably between 1% and 10% by weight of the composition.

Silicone oils may be divided into the volatile and non-volatile variety. The term "volatile" as used herein refers to those materials which have a measurable vapor pressure at ambient temperature. Volatile silicone oils are preferably chosen from cyclic (cyclomethicone) or linear polydimethylsiloxanes containing from 3 to 9, preferably from 4 to 5, silicon atoms.

Non-volatile silicone oils useful as an emollient material include polyalkyl siloxanes, polyalkylaryl siloxanes and polyether siloxane copolymers. The essentially non-volatile polyalkyl siloxanes useful herein include, for example, polydimethyl siloxanes with viscosities of from about $5 \times 10^{-6}$ to $0.1 \, m^2/s$ at 25°C. Among the preferred non-volatile emollients useful in the present compositions are the polydimethyl siloxanes having viscosities from about $1 \times 10^{-5}$ to about $4 \times 10^{-4}$ $m^2/s$ at 25°C.

Another class of non-volatile silicones are emulsifying and non-emulsifying silicone elastomers. Representative of this category is Dimethicone/Vinyl Dimethicone Crosspolymer available as Dow Corning 9040, General Electric SFE 839, and Shin-Etsu KSG-18. Silicone waxes such as Silwax WS-L (Dimethicone Copolyol Laurate) may also be useful.

Among the ester emollients are:

Alkenyl or alkyl esters of fatty acids having 10 to 20 carbon atoms such as isoarachidyl neopentanoate, isononyl isonanonoate, oleyl myristate, oleyl stearate, and oleyl oleate;

Ether-esters such as fatty acid esters of ethoxylated fatty alcohols;

Polyhydric alcohol esters such as ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono- and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters and polyoxyethylene sorbitan fatty acid esters. Particularly useful are pentaerythritol, trimethylolpropane and neopentyl glycol esters of $C_{1-30}$ alcohols;

Wax esters such as beeswax, spermaceti wax and tribehenin wax;

Sterols esters, of which cholesterol fatty acid esters are examples thereof; and

Sugar ester of fatty acids such as sucrose polybehenate and sucrose polycottonseedate.

Hydrocarbons which are suitable cosmetically acceptable carriers include petrolatum, mineral oil, $C_{11-13}$ iso-paraffins, polyalphaolefins, and especially isohexadecane (available commercially as Permethyl 101A from Presperse Inc.).

Fatty acids having from 10 to 30 carbon atoms may also be suitable as cosmetically acceptable carriers. Illustrative of this category are pelargonic, lauric, myristic, palmitic, stearic, isostearic, hydroxystearic, oleic, linoleic, ricinoleic, arachidic, behenic and erucic acids.

Fatty alcohols having from 10 to 30 carbon atoms are another useful category of cosmetically acceptable carrier. Illustrative of this category are stearyl alcohol, lauryl alcohol, myristyl alcohol and cetyl alcohol.

Humectants of the polyhydric alcohol-type can be employed as cosmetically acceptable carriers. Typical polyhydric alcohols include glycerin (glycerol), polyalkylene glycols and more preferably alkylene polyols and their derivatives, including propylene glycol, dipropylene glycol, polypropylene glycol, polyethylene glycol and derivatives thereof, sorbitol, raffinose, glucose, trehalose, hydroxypropyl sorbitol, hexylene glycol, 1,3-butylene glycol, isoprene glycol, 1,2,6-hexanetriol, ethoxylated glycerol, propoxylated glycerol and mixtures thereof. The amount of humectant may range anywhere from 0.5% to 50%, preferably between 1% and 15% by weight of the composition.

Thickeners can be utilized as part of the cosmetically acceptable carrier of compositions according to the present invention. Typical thickeners include crosslinked acrylates (e.g. Carbopol 982®), hydrophobically-modified acrylates (e.g. Carbopol 1382®), cellulosic derivatives and natural gums. Among useful cellulosic derivatives are sodium carboxymethylcellulose, hydroxypropyl methocellulose, hydroxypropyl cellulose, hydroxyethyl cellulose, ethyl cellulose and hydroxymethyl cellulose. Natural gums suitable for the present invention include guar, xanthan, sclerotium, carrageenan, pectin and combinations of these gums. Inorganics may also be utilized as thickeners, particularly clays such as bentonites and hectorites, fumed silicas, and silicates such as magnesium aluminum silicate (Veegum®). Amounts of the thickener may range from 0.0001% to 5%, usually from 0.001 % to 1%, optimally from 0.01 % to 0.5% by weight of the composition.

Emulsifiers may also be incorporate into compositions of this invention. These emulsifiers may be anionic, nonionic, cationic, amphoteric and combinations thereof. Useful nonionic type emulsifiers include the $C_{10-20}$ fatty alcohol or acid hydrophobes condensed with from 2 to 100 moles of ethylene oxide or propylene oxide per mole of hydrophobe; $C_{2-10}$ alkyl phenols condensed with from 2 to 20 moles of alkylene oxide; mono- and di-fatty acid esters of ethylene glycol; fatty acid monoglyceride; sorbitan, mono- and di-$C_{8-20}$ fatty acids; block copolymers (ethylene oxide/propylene oxide); and polyoxyethylene sorbitan as well as combinations thereof. Alkyl polyglycosides and saccharide fatty amides (e.g. methyl gluconamides) are also suitable nonionic emulsifiers.

Particularly preferred as an emulsifier is a hydrogenated castor wax alkoxylated with 40 moles ethylene oxide (available commercially as Cremophore RH-40®).

Mild emulsifiers of the anionic and amphoteric type may also be employed. Particularly preferred anionic examples include $C_{8-20}$ acyl isethionate salts, lauroamphoacetate salts and sarcosinate salts. Preferred amphoterics include cocamidopropylbetaine and dimethylbetaine.

Advantageously it may be desirable to avoid the presence of any emulsifiers or surfactants because these actives tend to break the lipid barrier of the skin causing irritation. They may also interfere with color stability.

Amounts of the emulsifiers may range from 0.05% to 20%, preferably from 0.1 % to 5%, and more preferably from 0.5% to 0.8%. In a particularly preferred embodiment, the emulsifiers are present at less than 0.1% by weight of the composition.

Sunscreen actives may also be included in compositions of the present invention. Particularly preferred are such materials as ethylhexyl p-methoxycinnamate (available as Parsol MCX®), Avobenzene (available as Parsol 1789®) and Benzophenone-3 (also known as Oxybenzone). Inorganic sunscreen actives may be employed such as microfine titanium dioxide, zinc oxide, polyethylene and various other polymers. Amounts of the sunscreen agents when present may generally range from 0.1 % to 10%, preferably from 0.5% to 5%, optimally from 1% to 2% by weight of the composition.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, imidazolidinyl urea, sodium dehydroacetate and benzyl alcohol. The preservatives should be selected having regard for the use of the composition and possible incompatibilities between the preservatives and other ingredients in the emulsion. Preservatives are preferably employed in amounts ranging from 0.01% to 2% by weight of the composition.

Compositions of the present invention may include vitamins. Illustrative vitamins are Vitamin A (retinol), Vitamin $B_2$, Vitamin $B_6$, Vitamin C, Vitamin E and Biotin. Derivatives of the vitamins may also be employed. For instance, Vitamin C derivatives include ascorbyl tetraisopalmitate, magnesium ascorbyl phosphate and ascorbyl glycoside. Derivatives of Vitamin E include tocopheryl acetate, tocopheryl palmitate and tocopheryl linoleate. DL-panthenol and derivatives may also be employed. Total amount of vitamins when present in compositions according to the present invention may range from 0.001% to 10%, preferably from 0.01% to 1%, optimally from 0.1 % to 0.5% by weight of the composition.

Another type of useful substance can be that of an enzyme such as oxidases, proteases, lipases and combinations. Particularly preferred is superoxide dismutase (commercially available as Biocell SOD from the Brooks Company, USA).

Skin lightening compounds may be included in the compositions of the invention. Illustrative substances are placental extract, lactic acid, niacinamide, arbutin, kojic acid, ferulic acid, resorcinol and derivatives including 4-substituted resorcinols and combinations thereof. Amounts of these agents may range from 0.1 % to 10%, preferably from 0.5% to 2% by weight of the compositions.

Desquamation promoters may be present. Illustrative are the alpha-hydroxycarboxylic acids and beta-hydroxycarboxylic acids. The term "acid" is meant to include not only the free acid but also salts and $C_{1-30}$ alkyl or aryl esters thereof and lactones generated from removal of water to form cyclic or linear lactone structures. Representative acids are glycolic, lactic and malic acids. Salicylic acid is representative of the beta-hydroxycarboxylic acids. Amounts of these materials when present may range from 0.01 % to 8% by weight of the composition.

Also included may be such materials as lipoic acid, retinoxytrimethylsilane (available from Clariant Corp. under the Silcare 1 M-75 trademark), dehydroepiandrosterone (DHEA) and combinations thereof. Ceramides (including Ceramide 1, Ceramide 3, Ceramide 3B and Ceramide 6) as well as pseudoceramides may also be utilized for many compositions of the present invention. Amounts of these materials may range from 0.000001% to 10%, preferably from 0.0001 % to 1% by weight of the composition.

Colorants, fragrances, opacifiers and abrasives may also be included in compositions of the present invention. Each of these substances may range from 0.05% to 5%, preferably between 0.1 % and 3% by weight of the composition.

Compositions of the present invention may further include non-tea herbal extracts. Illustrative extracts include Scullcap, Nettle Root, *Swertia Japonica,* Fennel and *Aloe Vera* extracts. Amount of each of the extracts may range from 0.0001 % to 1%, preferably from 0.01 % to 0.5%, optimally from 0.05% to 0.2% by weight of a composition.

Anti-irritant agents may also be present including those of steviosides, alpha-bisabolol and glycyhrizzinate salts, each anti-irritant agent being present in amounts ranging from 0.0001% to 1%, preferably from 0.01% to 0.3% by weight of the composition.

A further necessary aspect of the present invention is that of a substrate. Preferably the substrate is a water-insoluble substance. By "water-insoluble" is meant the substrate does not dissolve in or readily break apart upon immersion in water.

A wide variety of materials can be used as the substrate. The following non-limiting characteristics are desirable: (I) sufficient wet strength for use, (ii) sufficient abrasivity, (iii) sufficient loft and porosity, (iv) sufficient thickness, (v) appropriate size, and (vi) nonreactive with components of the impregnating composition.

Non-limiting examples of suitable substrates which meet at least some of the above criteria include non-woven substrates, woven substrates, hydroentangled substrates, air entangled substrates. Preferred embodiments employ non-woven substrates since they are economical and readily available in a variety of materials. By non-woven is meant that the layer is comprised of fibers which are not woven into a fabric but rather are formed into a sheet, particularly a tissue. The fibers can either be random (i.e., randomly aligned) or they can be carded (i.e. combed to be oriented in primarily one direction). Furthermore, the non-woven substrate can be composed of a combination of layers of random and carded fibers.

Non-woven substrates may be comprised of a variety of materials both natural and synthetic. By natural is meant that the materials are derived from plants, animals, insects or by-products. By synthetic is meant that the materials are obtained primarily from various man-made materials or from material that is usually a fibrous web comprising any of the common synthetic or natural textile-length fibers, or mixtures thereof.

Non-limiting examples of natural materials useful in the present invention are silk fibers, keratin fibers and cellulosic fibers. Non-limiting examples of keratin fibers include those selected from the group consisting of wool fibers, camel hair fibers. Non-limiting examples of cellulosic fibers include those selected from the group consisting of wood pulp fibers, cotton fibers, hemp fibers, jute fibers, flax fibers, and mixtures thereof. Wood pulp fibers are preferred.

Non-limiting examples of synthetic materials useful in the present invention include those selected from the group consisting of acetate fibers, acrylic fibers, cellulose ester fibers, modacrylic fibers, polyamide fibers, polyester fibers, polyolefin fibers, polyvinyl alcohol fibers, rayon fibers and mixtures thereof. Examples of some of these synthetic materials include acrylics such as Acrilan®, Creslan®, and the acrylonitrile-based fiber, Orlon®; cellulose ester fibers such as cellulose acetate, Arnel®, and Acele®; polyamides such as Nylons (e.g., Nylon 6, Nylon 66, Nylon 610); polyesters such as Fortrel®, Kodel®, and the polyethylene terephthalate fibers, Dacron®; polyolefins such as polypropylene, polyethylene; polyvinyl acetate fibers and mixtures thereof.

Non-woven substrates made from natural materials consist of webs or sheets most commonly formed on a fine wire screen from a liquid suspension of the fibers.

Substrates made from natural materials useful in the present invention can be obtained from a wide variety of commercial sources. Non-limiting examples of suitable commercially available paper layers useful herein include Airtex® (an embossed air-laid cellulosic layer available from James River Corporation, Green Bay, WI); and Walkisoft® (an embossed air-laid cellulosic available from Walkisoft U.S.A., Mount Holly, NC).

Non-woven substrates made from synthetic materials useful in the present invention can also be obtained from a wide variety of commercial sources. Non-limiting examples of suitable non-woven layer materials useful herein include HEF 40-047 (an apertured hydroentangled material containing about 50% rayon and 50% polyester available from Veratec, Inc., Walpole, MA); HEF 140-102 (an apertured hydroentangled material containing about 50% rayon and 50% polyester available from Veratec, Inc., Walpole, MA); Novenet® 149-191 (a thermo-bonded grid patterned material containing about 69% rayon, about 25% polypropylene and about 6% cotton available from Veratec, Inc., Walpole, MA); HEF Nubtex® 149-801 (a nubbed, apertured hydroentangled material, containing about 100% polyester available from Veratec, Inc. Walpole, MA); Keybak® 951V (a dry formed apertured material, containing about 75% rayon and about 25% acrylic fibers available from Chicopee Corporation, New Brunswick, NJ); Keybak® 1368 (an apertured material, containing about 75% rayon and about 5% polyester available from Chicopee Corporation, New Brunswick, NJ); Duralace® 1236 (an apertured, hydroentangled material, containing about 100% rayon available from Chicopee Corporation, New Brunswick, NJ); Duralace® 5904 (an apertured, hydroentangled material, containing about 100% polyester available from Chicopee Corporation, New Brunswick, NJ); and Sontaro® 8868 (a hydroentangled material, containing about 50% cellulose and about 50% polyester available from Dupont Chemical Corp).

Most preferred as a towelette for purposes of this invention are non-woven substrates, especially blends of rayon/polyester at ratios of 10:90 to 90:10, preferably ratios of 20:80 to 80:20, optimally 40:60 to 60:40 by weight. A most useful towelette is a 70:30 rayon/polyester non-woven wipe article.

The substrate can be made into a wide variety of shapes and forms. Generally the substrate is in single use towelette form and may be folded. Advantageously the size of the towelette may range in length from 10 cm to 40 cm, preferably from 15 cm to 30 cm, optimally from 18 cm to 24 cm. The width of the towelette may range from 8 cm to 30 cm, preferably from 10 cm to 25 cm, optimally from 15 cm to 20 cm.

Anywhere from 5 to 100, preferably from 10 to 50 single towelettes may be stored within a dispensing pouch, preferably a moisture impermeable pouch. During storage and between dispensing, the pouch is resealable, usually via an adhesive strip covering a dispensing opening. Single towelette containing pouches may also be employed.

The substrates of the present invention can comprise two or more layers, each having a different texture and abrasiveness. The differing textures can result from the use of different combinations of materials or from the use of a substrate having a more abrasive side for exfoliation and a softer, absorbent side for gentle cleansing. In addition, separate layers of the substrate can be manufactured to have different color, thereby helping the user to further distinguish the surfaces.

The water-insoluble substrate and cosmetic composition may be present in weight ratios of respectively 20:1 to 1:4, preferably from 3:1 to 1:1.5 and optimally 1:1.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

The following examples will more fully illustrate the embodiments of this invention. All parts, percentages and proportions referred to herein and in the appended claims are by weight unless otherwise illustrated.

EXAMPLE

A series of experiments were conducted to determine efficacy of tea extract and different types of copolymers on minimizing color change of a non-woven towelette formulated as a make-up remover.

Colorimetry was utilized for the evaluations. By way of background, colorimeters are devices that utilize red, green and blue filters to emulate the response of the human eye to light and color. A Gretag MacBeth Color-Eye 7000A utilized for the present experiments employs the CIE 1976 Lab color space method for measuring object color.

In this method, color space can be visualized as a three dimensional space, where the location of any color in the space is determined by its color coordinates: L, a, and b. L is a measured of the lightness of a sample, and ranges from 0 (black) to 100 (white). The quantities a and b (called opponent-type coordinates), define the degree of redness (positive a*) or greenness (negative a), yellowness (positive b) or blueness (negative b). These coordinates (a and b) approach zero for neutral colors (white, grays, and blacks). The higher the values for a and b, the more saturated the color.

The difference in absolute color coordinates between a trial and a standard quantifies the Color Difference (D) between the two.

Standard (1)=L1 x a1 x b1
Trial (2) = L2 x a2 x b2
D(L) = L2 - L1
D(a) = a2 - a1
D(b) = b2 - a1

These differences are called Deltas. Delta (DE) shows the magnitude of a total color difference and is defined as:

$$DE = [(DL)^2 + (Da)^2 + (Db)^2]1/2$$

The test procedure involved coating a cosmetic composition onto a non-woven substrate (70% rayon/30% polyester (Polymer Group Inc. and Green Bay Non-Wovens)). Measurements were taken on the Gretag MacBeth Color-Eye 7000A instrument. The composition impregnated samples were placed in a temperature controlled chamber operating under the sequence: 4°C, 25°C, 41 °C and 51 °C; and measurements taken at intervals of: 7 days, 14 days, 30 days, 60 days and 90 days. The samples were measured in the reflectance mode. The standards were the composition impregnated samples before testing. The results given in Table II are the average values of those measured at the various time intervals for each test temperature.
Table I lists formulation ingredients for the various tested samples.

TABLE I

| INGREDIENTS | Sample (Weight %) | | | | | | |
|---|---|---|---|---|---|---|---|
| | A* | B* | C* | D* | E* | F* | G |
| Water | 96.48 | 96.38 | 96.58 | 96.215 | 96.28 | 96.28 | 96.215 |
| Glycerin | 1 | 1 | 1 | 1 | 1 | 1 | 1 |
| Hexylene Glycol | 2 | 2 | 2 | 2 | 2 | 2 | 2 |
| Roman Chamomile Extract | 0.1 | 0.1 | 0.1 | 0.1 | 0.1 | 0.1 | 0.1 |
| Trimethylglycine | 0.01 | 0.01 | 0.01 | 0.01 | 0.01 | 0.01 | 0.01 |
| Raffinose | 0.01 | 0.01 | 0.01 | 0.01 | 0.01 | 0.01 | 0.01 |
| Sodium PCA | 0.1 | 0.1 | 0.1 | 0.1 | 0.1 | 0.1 | 0.1 |
| Glydant Plus Liquid® | 0.2 | 0.2 | 0.2 | 0.2 | 0.2 | 0.2 | 0.2 |
| Tea Extract Blend | -- | 0.2 | -- | 0.2 | 0.2 | 0.2 | 0.2 |
| Sodium Hydroxide (25% water) | -- | -- | -- | 0.065 | -- | -- | 0.065 |
| Pemulen® | -- | -- | -- | 0.1 | -- | -- | -- |
| Simulgel A® | -- | -- | -- | -- | 0.1 | -- | -- |
| Aristoflex AVC® | -- | -- | -- | -- | -- | 0.1 | -- |
| Stabylen 30® | 0.1 | -- | -- | -- | -- | -- | 0.1 |
| Tea Extract Blend is: (82-85% Distilled Water, 8-12% Butylene Glycol, 1-3% Camellia Sinensis Leaf Extract (green tea), 1-3% White Tea Leaf Extract, 0.09-1.1% Cyclopia Intermedia (Honeybush) Leaf, 0.09-0.11% Phenoxyethanol). Pemulen® is: Acrylates/C10-30 Alkyl Acrylate Crosspolymer Simulgel A® is: Ammonium Polyacrylate/isohexadecane/PEG-40 Castor Oil Aristoflex AVC® is: Ammonium Acryloyldimethyltaurate/VP Copolymer Stabylen 30® is: AcrylatesNinyl Isodecanoate Crosspolymer * comparative example | | | | | | | |

TABLE II

| Colorimeter Results | | | | | | | |
|---|---|---|---|---|---|---|---|
| Temperature (°C) | Sample No. (Mean DE) | | | | | | |
| | A* | B* | C* | D* | E* | F* | G |
| 4C | 4.237 | 4.7 | 4.733 | 3.356 | 4.61 | 3.993 | 2.02 |
| 25C | 4.4 | 5.177 | 4.431 | 3.889 | 3.876 | 3.963 | 1.707 |
| 41C | 4.203 | 4.325 | 4.072 | 3.624 | 4.278 | 3.857 | 2.187 |
| 51C | 4.523 | 3.864 | 4.114 | 3.913 | 4.113 | 4.046 | 2.13 |
| * comparative example | | | | | | | |

Based on the results reported in Table II, it is seen that the combination for Stabylen 30® and tea extract enhances color stability of the non-woven wipe. The absence of one of these ingredients or of both caused the DE to be higher (greater color instability) than the formula including both ingredients (Sample G). Other copolymers such as Pemulen®, Simulgel A® and Aristoflex ARC® were each less effective than the Stabylen 30®.

**Claims**

1. A cosmetic towelette product comprising:

    (i) a water-insoluble substrate;
    (ii) a cosmetic composition in contact with the substrate, the composition comprising:

    (a) from 0.001 % to 10% by weight of the composition of a co-polymer comprised from monomers one of which is a vinyl ester of a $C_{3-20}$ acid;
    (b) from 0.000001% to 2% by weight of the composition of a tea extract; and
    (c) from 1% to 99.9% by weight of the composition of a cosmetically acceptable carrier.

2. A towelette according to claim 1 wherein the composition further comprises less than 0.1% of an emulsifier.

3. A towelette according to claim 1 or claim 2 wherein the substrate is a blend of rayon and polyester in a weight ratio of 10:90 to 90:10.

4. A towelette according to any one of the preceding claims wherein the tea extract is selected from the group consisting of *Camellia Sinensis* green tea leaf extract, white tea leaf extract, black tea leaf extract and combinations thereof.

5. A towelette according to any one of claims 1 to 3 wherein the tea extract is a combination of green tea, white tea and black tea extracts.

6. A towelette according to any one of the preceding claims wherein other monomers forming the copolymer are selected from the group consisting of acrylic acid, methacrylic acid, ethylacrylate, methyl acrylate, methyl methacrylate, ethyl methacrylate, butyl acrylate, hydroxypropyl methacrylate, hydroxyethyl acrylate, divinyl benzene, vinyl caprolactam, vinyl acetate , maleic acid or anhydride, acrylamide, methacrylamide, $C_{10-30}$ ester of acrylic or methacrylic acids, acryloyldimethyltaurate, styrene, methyl styrene, isoprene, butene, isohexadecane, vinyl chloride, vinyl pyrrolidine, vinyl methyl ether, vinylformamide and combinations thereof, as well as salts of monomers having carboxylate groups.

7. A towelette according to any one of claims 1 to 5 wherein the copolymer is Acrylates/Vinyl Isodecanoate Crosspolymer (INCI name).

**EP 1 893 293 B1**

**Patentansprüche**

1. Kosmetiktuchprodukt, umfassend:

   (i) ein wasserunlösliches Substrat;
   (ii) eine Kosmetikzusammensetzung in Kontakt mit dem Substrat, wobei die Zusammensetzung umfasst:

   (a) 0,001 Gew.-% bis 10 Gew.-%, bezogen auf die Zusammensetzung, eines Copolymers, das aus Monomeren besteht, von denen eines ein Vinylester einer $C_{3-20}$-Säure ist;
   (b) 0,000001 Gew.-% bis 2 Gew.-%, bezogen auf die Zusammensetzung, eines Teeextraktes und
   (c) 1 Gew.-% bis 99,9 Gew.-%, bezogen auf die Zusammensetzung, eines kosmetisch verträglichen Trägers.

2. Tuch nach Anspruch 1, wobei die Zusammensetzung außerdem weniger als 0,1 % eines Emulgators umfasst.

3. Tuch nach Anspruch 1 oder Anspruch 2, wobei das Substrat eine Mischung aus Rayon und Polyester in einem Gewichtsverhältnis von 10:90 bis 90: 10 ist.

4. Tuch nach einem der vorangehenden Ansprüche, wobei der Teeextrakt aus der Gruppe, bestehend aus dem grünen Tee-Blätterexrakt, dem weißen Tee-Blätterextrakt, dem schwarzen Tee-Blätterextrakt von *Camellia Sinensis* und Kombinationen daraus, ausgewählt ist.

5. Tuch nach einem der Ansprüche 1 bis 3, wobei der Teeextrakt eine Kombination von Extrakten von grünem Tee, weißem Tee und schwarzem Tee ist.

6. Tuch nach einem der vorangehenden Ansprüche, wobei andere Monomere, die das Copolymer bilden, aus der Gruppe, bestehend aus Acrylsäure, Methacrylsäure, Ethylacrylat, Methylacrylat, Methylmethacrylat, Ethylmethacrylat, Butylacrylat, Hydroxypropylmethacrylat, Hydroxyethylacrylat, Divinylbenzol, Vinylcaprolactam, Vinylacetat, Maleinsäure oder -anhydrid, Acrylamid, Methacrylamid, $C_{10-30}$-Ester von Acryl- oder Methacrylsäure, Acryloyldimethyltaurat, Styrol, Methylstyrol, Isopren, Buten, Isohexadecan, Vinylchlorid, Vinylpyrrolidin, Vinylmethylether, Vinylformamid und Kombinationen davon sowie Salzen von Monomeren, die Carboxylatgruppen haben, ausgewählt sind.

7. Tuch nach einem der Ansprüche 1 bis 5, wobei das Copolymer Acrylates/Vinyl Isodecanoate Crosspolymer (INCI-Name) ist.

**Revendications**

1. Produit de lingette cosmétique, qui comprend :

   (i) un substrat insoluble dans l'eau ;
   (ii) une composition cosmétique en contact avec le substrat, la composition comprenant :

   (a) de 0,001 % à 10 % en poids de la composition d'un copolymère composé de monomères dont l'un est un ester vinylique d'un acide en $C_{3-20}$ ;
   (b) de 0,000001 % à 2 % en poids de la composition d'un extrait de thé ; et
   (c) de 1% à 99,9 % en poids de la composition d'un vecteur acceptable sur le plan cosmétique.

2. Lingette selon la revendication 1, dans laquelle la composition comprend en outre moins de 0,1 % d'un émulsifiant.

3. Lingette selon la revendication 1 ou la revendication 2, dans laquelle le substrat est un mélange de rayonne et de polyester en un rapport en poids de 10/90 à 90/10.

4. Lingette selon l'une quelconque des revendications précédentes, dans laquelle l'extrait de thé est choisi dans le groupe constitué par un extrait de feuilles de thé vert *Camellia Sinensis*, un extrait de feuilles de thé blanc, un extrait de feuilles de thé noir et les combinaisons de ceux-ci.

5. Lingette selon l'une quelconque des revendications 1 à 3, dans laquelle l'extrait de thé est une combinaison d'extraits de thé vert, de thé blanc et de thé noir.

**6.** Lingette selon l'une quelconque des revendications précédentes, dans laquelle les autres monomères formant le copolymère sont choisis dans le groupe constitué par l'acide acrylique, l'acide méthacrylique, l'acrylate d'éthyle, l'acrylate de méthyle, le méthacrylate de méthyle, le méthacrylate d'éthyle, l'acrylate de butyle, le méthacrylate d'hydroxypropyle, l'acrylate d'hydroxyéthyle, le divinyl benzène, le vinyl caprolactame, l'acétate de vinyle, l'acide ou l'anhydride maléique, l'acrylamide, le méthacrylamide, un ester en $C_{10-30}$ d'acides acryliques ou méthacryliques, l'acryloyldiméthyltaurate, le styrène, le méthyl styrène, l'isoprène, le butène, l'isohexadécane, le chlorure de vinyle, la vinylpyrrolidine, le méthyl éther de vinyle, le vinylformamide et les combinaisons de ceux-ci, ainsi que les sels de monomères ayant des groupes carboxylate.

**7.** Lingette selon l'une quelconque des revendications 1 à 5, dans laquelle le copolymère est un polymère réticulé d'acrylates/isodécanoate de vinyle (dénomination INCI Acrylates/Vinyl Isodecanoate Crosspolymer).

**EP 1 893 293 B1**

**Patent documents cited in the description**

- US 6491937 B1, Slavtcheff

- US 6294182 B1, Znaiden